Europäisches Patentamt

(19) **European Patent Office**

Office européen des brevets

(11) Veröffentlichungsnummer: **0 033 929**
**B1**

(12) **EUROPÄISCHE PATENTSCHRIFT**

(45) Veröffentlichungstag der Patentschrift:
06.07.83

(51) Int. Cl.³: **C 07 C 69/18,** C 07 C 67/08

(21) Anmeldenummer: 81100716.0

(22) Anmeldetag: 02.02.81

(54) Verfahren zur kontinuierlichen Herstellung von Triacetin.

(30) Priorität: 08.02.80 DE 3004660

(43) Veröffentlichungstag der Anmeldung:
19.08.81 Patentblatt 81/33

(45) Bekanntmachung des Hinweises auf die Patenterteilung:
06.07.83 Patentblatt 83/27

(84) Benannte Vertragsstaaten:
AT BE CH DE FR GB IT LI NL SE

(56) Entgegenhaltungen:
DD-A-134 867
DE-A-2 503 195
DE-B-2 022 011

(73) Patentinhaber: Henkel Kommanditgesellschaft auf
Aktien, Postfach 1100 Henkelstrasse 67,
D-4000 Düsseldorf 1 (DE)

(72) Erfinder: Bremus, Norbert, Opladener Strasse 144,
D-4018 Langenfeld (DE)
Erfinder: Dieckelmann, Gerhard, Dr., Luisenstrasse 19,
D-4010 Hilden (DE)
Erfinder: Jeromin, Lutz, Dr., Am Nettchesfeld 41,
D-4000 Düsseidorf 13 (DE)
Erfinder: Rupilius, Wolfgang, Dr., Am Rittersberg 30,
D-4000 Düsseldorf-Urdenbach (DE)
Erfinder: Schütt, Hartwig, Dr., Haydnstrasse 50,
D-4000 Düsseldorf-Benrath (DE)

## Verfahren zur kontinuierlichen Herstellung von Triacetin

Die Erfindung betrifft ein kontinuierliches Verfahren zur Herstellung von Triacetin aus Glycerin, Essigsäure und Essigsäureanhydrid.

Aus der DE-PS 347 897 ist ein diskontinuierliches Verfahren zur Herstellung von Triacetin bekannt, bei dem man Glycerin mit Essigsäureanhydrid im Molverhältnis 1 : 3 bei 130 bis 135°C miteinander umsetzt. Dabei entstehen pro Mol umgesetztem Glycerin 3 Mol Essigsäure, für die im allgemeinen kaum eine Verwendungsmöglichkeit besteht, weshalb diese Methode für die großtechnische Herstellung von Triacetin praktisch nicht in Frage kommt.

Weiterhin sind — wie aus Seifen — Öle — Fette — Wachse 1962. S. 597 bis 602 hervorgeht — diskontinuierliche Verfahren zur Herstellung von Triacetin aus Glycerin und Essigsäure bekannt. Da bei dieser Reaktion das Gleichgewicht auf der Seite der Ausgangsstoffe liegt, ist man hier gezwungen, das bei der Veresterungsreaktion entstehende Wasser kontinuierlich aus dem Reaktionsgemisch zu entfernen, um mit rationellen Essigsäuremengen auszukommen. Diese Verfahren erfordern lange Reaktionszeigen und sind deshalb mit einem hohen Energieverbrauch und einer schlechten Ausnutzung der Reaktorkapazität verbunden.

Wie man aus Seifen—Öle—Fette—Wachse 1962, S. 598 entnehmen kann, läßt sich die Veresterungsreaktion besonders im niedrigen Temperaturbereich von 100 bis 160°C durch saure Katalysatoren beschleunigen. Damit sich der Katalysator nicht in der Dampfphase anreichert, muß er einen niedrigeren Dampfdruck als das Glycerin aufweisen.

Es wurde nun gefunden, daß man die Nachteile der bekannten Verfahren weitgehend ausschalten kann, wenn man das nachstehend beschriebene kontinuierliche Verfahren zur Herstellung von Triacetin benutzt.

Gegenstand der Erfindung ist ein Verfahren zur Herstellung von Triacetin durch Umsetzen von Glycerin mit Essigsäure und Essigsäureanhydrid bei erhöhter Temperatur und gegebenenfalls in Anwesenheit von Katalysatoren, das dadurch gekennzeichnet ist, daß man die Reaktionspartner in kontinuierlicher Arbeitsweise in Gegenstrom miteinander umsetzt, wobei man in einer mit einer Mehrzahl von Böden ausgestatteten Veresterungskolonne bei einem Druck von 0,2 bis 30 bar und einer Temperatur von 100 bis 250°C flüssiges Glycerin einem aufsteigenden Strom von überhitztem Essigsäuredampf entgegenführt, wobei die Verweilzeit des flüssigen Reaktionsgemisches mindestens eine Stunde beträgt, und dem herabströmenden Reaktionsgemisch beim Erreichen einer OH-Zahl von weniger als 600 auf dem entsprechenden Boden der Reaktionskolonne oder in einem entsprechend ausgelegten Nachreaktor soviel Essigsäureanhydrid zuführt, daß das an dieser Stelle in der flüssigen Reaktionsphase gelöste Wasser quantitativ zu Essigsäure und das vorhandene Mono- und Diacetin quantitativ zu Triacetin abreagieren können.

Wird das Verfahren in Abwesenheit von Katalysatoren durchgeführt, so liegt der Reaktionsdruck im Bereich von 3 bis 30 bar und die Reaktionstemperatur im Bereich von 180 bis 250°C. Bei Anwesenheit von Katalysatoren beträgt der Reaktionsdruck 0,2 bis 3 bar und die Reaktionstemperatur 100 bis 180°C.

Für die Durchführung des erfindungsgemäßen Verfahrens kommen saure Veresterungskatalysatoren in Betracht, insbesondere konz. Schwefelsäure, Trifluoressigsäure, Kaliumhydrogensulfat, Aluminiumsulfat oder Zinkacetat. Vorzugsweise wird p-Toluolsulfonsäure als Katalysator eingesetzt, bezogen auf eingesetztes Glycerin beträgt die Katalysatormenge 0,01 bis 0,5 Gewichtsprozent.

Bei der Durchführung des erfindungsgemäßen Verfahrens wird bevorzugt Glycerin mit einer OH-Zahl von 1828 eingesetzt. Essigsäure wird in der Regel in einem molaren Verhältnis zum Glycerin von 2,5 : 1 bis 5 : 1 eingesetzt.

Die Menge des Essigsäureanhydrids, das dem Reaktionsgemisch im unteren Kolonnendrittel oder im Nachreaktor zugeführt wird, beträgt in der Regel 0,1 bis 1,5 Mol pro Mol insgesamt umzusetzenden Glycerin.

Die Herstellung von Triacetin nach dem erfindungsgemäßen Verfahren erfolgt zweckmäßigerweise in der nachstehend beschriebenen Vorrichtung zur Durchführung von Flüssigkeits-Dampf-Reaktionen. Die Vorrichtung besteht aus einer Reaktionskolonne mit aufgesetzter Verstärkungskolonne, Wärmeaustauschern, Lagerbehältern, Förderpumpen und verbindenden Rohrleitungen.

Die Reaktionskolonne ist als Glockenbodenkolonne mit mindestens 20 Doppelglockenböden ausgebildet. Die innere Glocke ist hierbei zur Einstellung des trockenen Druckverlustes und zur Abtauchung des Zulaufrohres des darüberliegenden Bodens beim Anfahren der Kolonne erforderlich. Die äußere Glocke dient zur Verteilung des Dampfes in der Flüssigkeit und schafft die Stoffaustauschfläche. Die gesamte von den Glocken auf einem Boden eingenommene Fläche beträgt 10 bis 30% der zwischen dem Zu- und Ablaufwehr liegenden Fläche. Das Verhältnis der Durchtrittsfläche des Dampfes durch die auf den Boden aufgesetzten Glocken beträgt, bezogen auf den freien Kolonnenquerschnitt, 0,3 bis 3%. Die Dampfdurchtrittsfläche ist auf mindestens 4 Bohrungen pro Glocke verteilt. Die Bohrungen haben einen Durchmesser von 2 bis 5 mm und befinden sich auf dem Umfang der Glocken in 5 bis 20 mm Höhe über dem Boden. Die Höhe des Auslaufrohres beträgt mindestens 80 mm.

Durch diese Ausgestaltung der Glockenbodenkolonne erreicht man bei niederer Dampfbelastung einen extrem hohen Flüssigkeitsstand auf den Böden, wodurch die für die Durchführung der Veresterung erforderliche lange Verweilzeit erreicht wird. Die Konstruktion und Anordnung der Glocken bewirkt eine gleichmäßige Verteilung der Dampfphase über den wirksamen Kolonnenquerschnitt, dämpft pulsierende Flüssigkeitsströmungen und erteilt dem Dampf beim Austritt aus der Glocke eine hohe Strömungsgeschwindigkeit. Es empfiehlt sich, die auf die Dampfdurchtrittsfläche bezogene vergleichbare Luftgeschwindigkeit etwa 4 bis 30 m/s, zweckmäßig 15 m/s betragen zu lassen, um die Vermischung der Reaktionspartner zu fördern.

Eine zweckmäßige Ausgestaltung der Durchströmungsquerschnitte für den Dampf durch die Glocken besteht darin, daß anstelle der kreisförmigen Bohrungen in der Glocke in Bodennähe mindestens 4 senkrecht stehende Schlitze von 2 bis 5 mm Breite und 5 bis 20 mm Höhe angebracht sind. Bei den hohen Flüssigkeitsständen auf den Böden und den niederen Dampfbelastungen werden die Schlitze jeweils nur zu einem geringen Teil mit Dampf beaufschlagt, während der übrigbleibende untere Teil der Schlitze von der auf dem Boden stehenden Flüssigkeit verschlossen wird. Gegenüber den Bohrungen haben die Schlitze den Vorteil, daß sie weniger leicht verstopfen. Außerdem steht bei größeren Dampfmengen jeweils automatisch eine größere Durchtrittsfläche zur Verfügung, da sich der Dampf gegebenenfalls einen größeren Querschnitt freiblasen kann. Weiterhin ist es zweckmäßig, daß die Zu- und Ablaufwehre mindestens die Höhe des den Flüssigkeitsstand bestimmenden Ablaufrohres aufweisen und in Bödennähe Schlitze besitzen, durch welche die Flüssigkeit zu- und abfließt.

Eine weitere zweckmäßige Ausgestaltung besteht darin, daß im Hals der inneren Glocke eine Blende mit einer Bohrung von 2 bis 15 mm vorhanden ist.

Bei größeren Kolonnenböden (Durchmesser größer als 300 mm) muß der auf dem Boden sich stärker ausbildende Flüssigkeitsgradient bei der Gestaltung der Glocken berücksichtigt werden. Eine gleichmäßige Verteilung des Dampfes auf alle Glocken des Bodens ist nämlich nur dann gewährleistet, wenn der sogenannte trockene Druckverlust des Bodens (der gemessen wird, wenn der Boden vom Dampf durchströmt und dabei nicht mit Flüssigkeit beaufschlagt wird) mindestens doppelt so groß ist wie der durch den Flüssigkeitsgradienten hervorgerufene hydrostatische Höhenunterschied zwischen Zu- und Ablauf. Der erforderliche trockene Druckverlust kann dadurch erzielt werden, daß eine Blende mit einer Bohrung von 2 bis 15 mm Durchmesser in den Hals der inneren Glocke eingebaut wird. Die effektiv erforderliche Blendengröße kann durch einfachen Versuch festgelegt werden. Der trockene Druckverlust kann auch durch einen hinreichend kleinen Abstand zwischen der Oberkante des Halses der inneren Glocke und dem oberen Teil der äußeren Glocke realisiert werden, der dann 1 bis 3 mm beträgt.

In den folgenden Figuren ist das erfindungsgemäße Verfahren und die Ausgestaltung der Reaktionsvorrichtung beispielhaft dargestellt. Es zeigen

Fig. 1 ein Schema des Verfahrens zur kontinuierlichen Herstellung von Triacetin,

Fig. 2a einen Längsschnitt durch einen Boden des Reaktors,

Fig. 2b eine Draufsicht auf einen Boden des Reaktors,

Fig. 2c eine vergrößerte Darstellung einer Glocke mit senkrecht stehenden Schlitzen,

Fig. 2d eine vergrößerte Darstellung einer Glocke mit einer im Glockenhals angebrachten Blende.

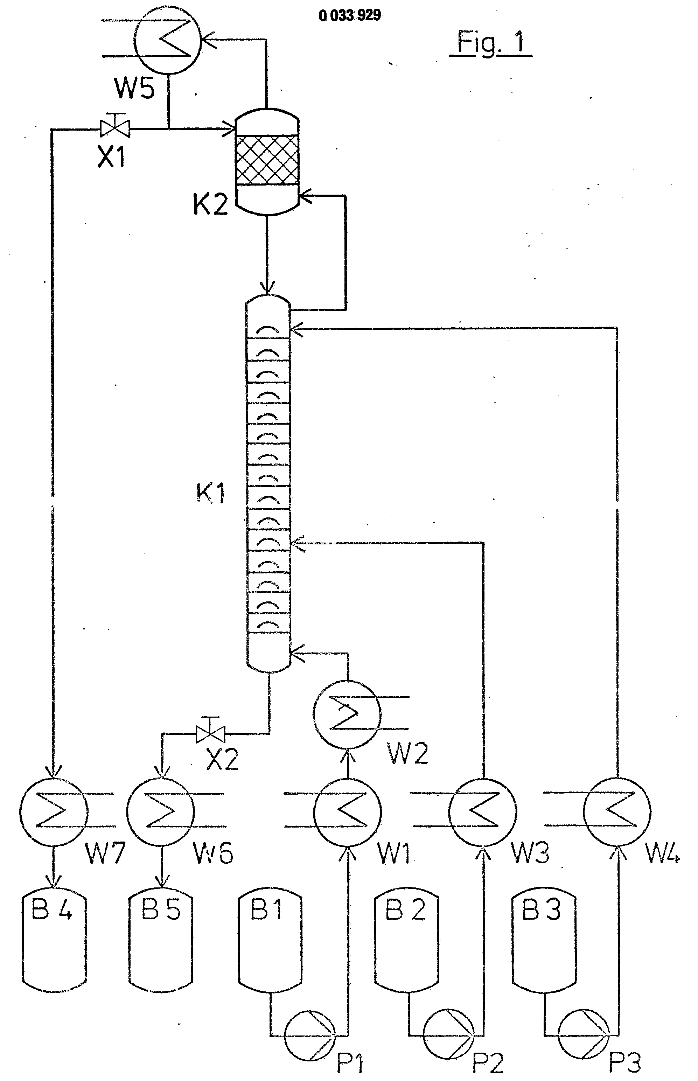

Nach dem Verfahrensschema (Fig. 1) wird Glycerin aus dem Vorratsbehälter B3 mit Hilfe der Pumpe P3 über den Wärmeaustauscher W4, wo es auf die Reaktionstemperatur erhitzt wird, unter dem vorgesehenen Reaktionsdruck auf den obersten Boden der Reaktionskolonne K1 gefördert. Essigsäure wird aus dem Vorratsbehälter B1 mittels der Pumpe P1 über den Verdampfer W1 und den Überhitzer W2 unter den untersten Boden der Reaktionskolonne K1 geführt.

Das auf den obersten Boden aufgegebene Glycerin, das gegebenenfalls den Katalysator enthält, durchströmt die Reaktionskolonne K1 im Gegenstrom zu dem aufsteigenden Essigsäuredampf. In Abhängigkeit von der Löslichkeit der Essigsäure in der flüssigen Phase auf den einzelnen Böden reagiert diese mit dem Glycerin oder mit dem bereits gebildeten Monoacetin und Diacetin. Bei diesen Reaktionen entsteht Wasser, das sich auf Grund des Phasengleichgewichts auf die Flüssigkeit und dem Dampf verteilt. Vom Kopf zum Sumpf der Kolonne nimmt zwar auf den Böden in der flüssigen Phase der Wassergehalt mit zunehmendem Umsatz (ausgedrückt z. B. durch die Abnahme der OH-Zahl) ab, jedoch wird er nicht Null und wirkt mit Wassergehalten bis zu 3 Gew.-% im flüssigen Reaktionsgemisch im Sumpf dem Ablauf der verschiedenen Veresterungsreaktionen, insbesondere der Triacetinbildung, entgegen. Dieser Wassergehalt würde schließlich dazu führen, daß die Veresterung über die Reaktionsgleichgewichtseinstellung zum Erliegen kommt. Um dies zu verhindern, wird erfindungsgemäß Essigsäureanhydrid aus dem Vorratsbehälter B2 mit Hilfe der Pumpe 2 über den Wärmeaustauscher W3, wo es auf die Reaktionstemperatur erhitzt wird, auf einen Boden im unteren Drittel der Kolonne K 1 eingespeist. Dieser Boden ist so gewählt, daß dort das von oben kommende Glycerin bereits einen Gesamtveresterungsgrad von circa 70% oder mehr, d. h. eine OH-Zahl von weniger als 600 aufweist. Es wird soviel flüssiges Essigsäureanhydrid zugeführt, daß das an dieser Stelle in der flüssigen Reaktionsphase gelöste Wasser quantitativ zu Essigsäure und das vorhandene Mono- und Diacetin zu Triacetin abreagieren können. Hierfür sind relativ geringe Mengen Essigsäureanhydrid im Bereich von 0,1 bis 1,5 Mol insgesamt umzusetzenden Glycerins erforderlich.

Alternativ kann die Nachacetylierung mit Essigsäureanhydrid in einen separaten Nachreaktor verlegt werden. In diesem Fall wird das Sumpfprodukt aus der Veresterungskolonne mit einer OH-Zahl von weniger als 600 zusammen mit Essigsäureanhydrid in den Nachacetylierungsreaktor eingespeist. Die bei der Acetylierung entstehende Essigsäure wird in diesem Fall in den Tank B1 zur weiteren Veresterung geleitet. Als Nachacetylierungsreaktoren kommen z. B. mit Füllkörpern gefüllte Rohrreaktoren oder Rührkesselkaskaden in Frage.

Die im Einzelfall zuzuführende Menge Essigsäureanhydrid hängt einerseits vom Veresterungsgrad, der wiederum abhängig ist von der Verweilzeit und damit von der Dimensionierung der benutzten Reaktionskolonne, andererseits von der Größe des molaren Verhältnisses von eingesetzter Essigsäure zum Glycerin ab.

Das im unteren Drittel der Reaktionskolonne K1 oder einem entsprechend dimensionierten Nachreaktor eingespeiste Essigsäureanhydrid reagiert mit dem in der flüssigen Reaktionsphase vorhandenen und dem beim weiteren Fortschreiten der Veresterungsreaktionen anfallenden Wasser und entfernt dieses aus dem Gleichgewicht unter gleichzeitiger Bildung von Essigsäure. Ein Teil des zugeführten Essigsäureanhydrids reagiert auch direkt mit den noch nicht veresterten OH-Gruppen des Glycerins unter Bildung von Essigsäure. Die bei diesen Reaktionen aus dem Essigsäureanhydrid entstehende Essigsäure steht für die Veresterung von Glycerin, Monoacetin und Diacetin zur Verfügung und wird bei der Berechnung der einzusetzenden Essigsäuregesamtmenge berücksichtigt.

Das am Kopf der Kolonne K1 anfallende Kopfprodukt wird als überhitzter Dampf abgezogen und in der Kolonne K2 rektifiziert. Glycerin, Monoacetin und gegebenenfalls im Kopfprodukt vorhandenes Diacetin werden in der Kolonne K2 kondensiert und fließen in den Reaktor K1 zurück.

Das abdestillierende Kopfprodukt, bestehend aus dem Reaktionswasser und bis zu 3 Gew.-% Essigsäure, wird im Wärmeaustauscher W5 kondensiert und unterkühlt, sodann über das Ventil X1 auf Atmosphärendruck entspannt und über einen weiteren Wärmeaustauscher W7 in den Lagerbehälter B4 abgezogen. Die im Destillat enthaltene Essigsäuremenge entspricht nur zu einem geringen Teil dem eingesetzten molaren Essigsäureüberschuß. Der zur Trennung in der Kolonne K2 aufgegebene Rücklauf ist so bemessen, daß die Überhitzungsenergie des der Kolonne K2 zuströmenden Dampfes und die Exothermie der Reaktion auf den obersten Boden der Kolonne K1 zur Verdampfung des Rücklaufs ausreichen.

Das Reaktionsprodukt wird aus dem Sumpf der Kolonne K1 bzw. aus dem Nachacetylierungsreaktor laufend abgezogen, im Wärmeaustauscher W6 abgekühlt, über das Ventil X2 entspannt und in den Behälter B5 geleitet. Es enthält neben Triacetin abhängig vom Umsatz und den gewählten Reaktionsbedingungen 20 bis 40 Gew.-% Essigsäure und bis 5 Gew.-% Essigsäureanhydrid den Rest des angewandten Essigsäure- bzw. Essigsäureanhydridüberschusses.

Bei richtiger Reaktionsführung sind in dem Reaktionsprodukt nur Spuren von Glycerin, Monoacetin, Diacetin und Wasser vorhanden, so daß die OH-Zahl normalerweise unter 1 liegt. Aus dem erhaltenen Rohprodukt kann reines Triacetin leicht durch Destillation gewonnen werden. Ein eventuell bei der Veresterung vorhandener Katalysator verbleibt dabei im Destillationsrückstand.

Erfindungsgemäß wird die Reaktionskolonne so ausgelegt, daß die in der Nachacetylierung entstehende Essigsäure bei der Veresterung des Glycerins umgesetzt wird, so daß im gesamten Prozeß keine Essigsäure im Überschuß entsteht. Am Kopf der Kolonne wird das bei der Reaktion entstehende Reaktionswasser abgezogen, wobei die Kolonne so dimensioniert ist, daß der in der Kolonne aufsteigende Essigsäuredampf weitgehend mit den OH-Gruppen des Glycerins abreagiert und oberhalb des obersten Bodens nur noch 1 bis 3 Gew.-% Essigsäure im Reaktionswasser enthalten sind. Nach einer Neutralisation kann das Kopfprodukt einer biologischen Kläranlage zugeführt werden, ohne vorher eine sehr kostspielige Abtrennung der Essigsäure aus dem Wasser vornehmen zu müssen, die bei höheren Essigsäurekonzentrationen im Kopfprodukt unumgänglich wird.

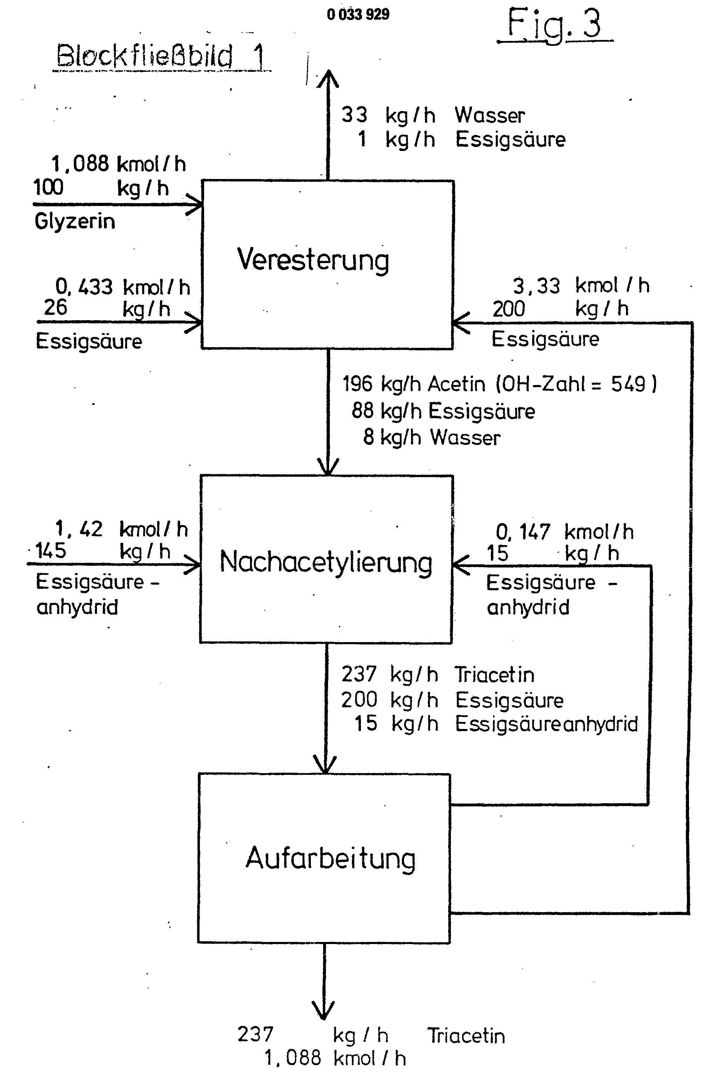

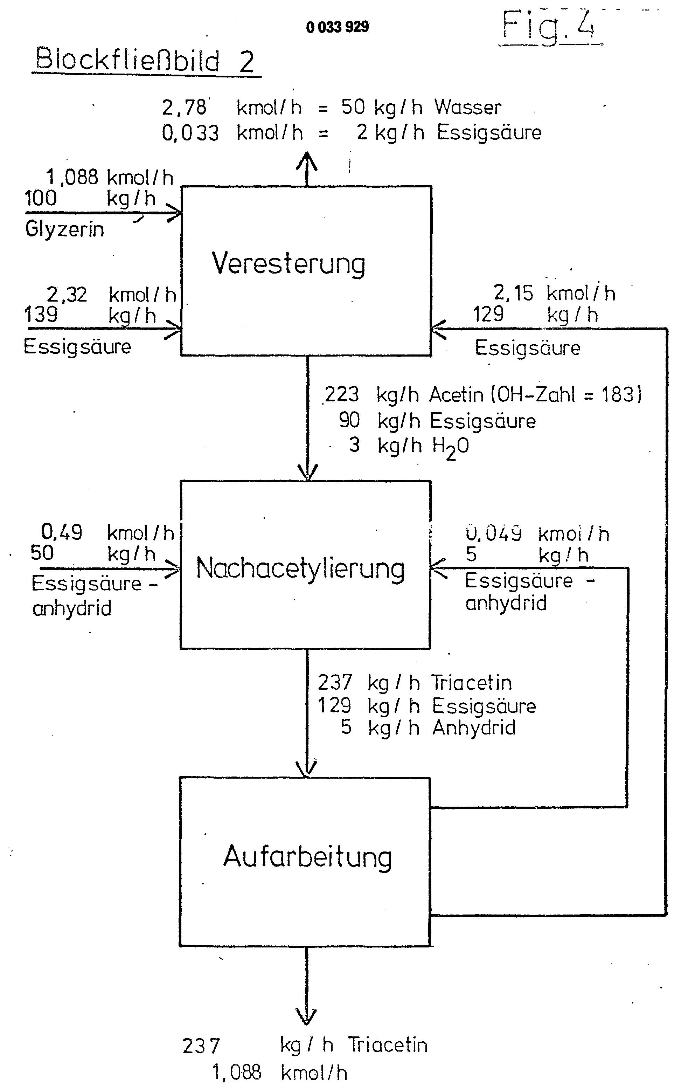

Zwischen der Aufteilung der Reaktion auf die Veresterungs- und Nachacetylierungsstufe bestehenden physikalisch-chemische Zusammenhänge, die im folgenden schematisch dargestellt sind. Auf den Blockfließbildern 1 und 2 (Fig. 3 und 4) sind die Zusammenhänge für einen 70%igen Umsatz, d. h. bis zu einer OH-Zahl von 549 und einem 90%igen Umsatz, d. h. bis zu einer OH-Zahl von 183, in der Veresterung dargestellt.

Blockfließbild 1 (Fig. 3): 70%iger Umsatz in der Veresterung.

Die Mengenangaben sind auf 100 kg/h Glycerin (OH-Zahl = 1828) bzw. 237 kg/h Triacetin bezogen. In der Veresterungskolonne wird das Glycerin mit 26 kg/h frischer und 200 kg/h Essigsäure, die in der Nachacetylierung aus Essigsäureanhydrid entstanden ist, zu 70% auf eine OH-Zahl von 549, d. h. bezogen auf das reine Glycerin bei einem Überschuß von 0,46 Mol Essigsäure, verestert. Die Kolonne ist hierbei erfindungsgemäß so zu dimensionieren, daß neben der Erzielung eines 70%igen Umsatzes das Reaktionswasser mit maximal 3% Essigsäure am Kopf der Kolonne abgezogen werden kann. Dieses Kopfprodukt kann dann nach einer Neutralisation z. B. in eine biologische Kläranlage geleitet werden, ohne daß eine kostspielige Abtrennung der Essigsäure aus dem Wasser vorgenommen werden muß, die bei höheren Essigsäurekonzentrationen im Kopfprodukt unumgänglich wäre.

Zur Erzielung eines 70%igen Umsatzes in der Veresterungskolonne bei gleichzeitiger Einhaltung einer Essigsäurekonzentration von weniger als drei Prozent im Kopfprodukt ist bei den in Frage kommenden Reaktionsbedingungen mit Temperaturen zwischen 180 und 250°C und Drücken von 3 bis 30 bar bei Abwesenheit von Katalysatoren und Temperaturen zwischen 100 und 180°C und Drücken von 0,5 bis 3 bar in Gegenwart von Katalysatoren eine Verweilzeit der Reaktanden auf den Kolonnenböden von mindestens einer Stunde, verteilt auf mindestens 20 Böden spezieller Konstruktion, erforderlich. Je nach gewählten Reaktionsbedingungen enthält das Sumpfprodukt der Veresterungskolonne neben dem Acetin mit einer OH-Zahl von 549 noch 20—40% Essigsäure und 2 bis 3% Wasser. Dieses Gemisch wird der Nachacetylierungsstufe zugeführt, die sowohl als Verlängerung der Veresterungskolonne als auch als getrennter Reaktor ausgeführt werden kann. Eine Verlängerung der Veresterungskolonne hat den Vorteil, daß die in der Nachacetylierung durch Reaktion des Anhydrids mit den restlichen OH-Gruppen und mit dem im Sumpfprodukt der Veresterungskolonne enthaltenen Reaktionswasser entstehende Essigsäure direkt ohne Aufarbeitung in der Veresterungskolonne umgesetzt wird, wozu der Nachacetylierungsteil der Kolonne allerdings mit einer Beheizung zur Verdampfung eines Teils der Essigsäure zu versehen ist, da die Reaktionsenthalpie der exothermen Nachacetylierung allein nicht für eine ausreichende Verdampfung der Essigsäure sorgt.

In der Nachacetylierung wird das Reaktionsgemisch aus der Veresterung mit 145 kg/h (1,31 Mol Anhydrid pro Mol Glycerin) frischem umzusetzenden und 15 kg/h im Kreis zu führenden Anhydrid als Überschuß zum Triacetin umgesetzt, d. h. 1,44 Mol Anhydrid pro Mol Glycerin. Das die Nebenacetylierungsstufe verlassende Triacetin enthält noch Essigsäure und Anhydrid, die in der Aufbereitung getrennt werden, wobei die Essigsäure in die Veresterung und das überschüssige Anhydrid in die Nachacetylierung zurückgeführt werden.

Die Nachacetylierung erfordert je nach Wahl der Reaktionsbedingungen von 100 bis 250°C und 0,5 bis 30 bar eine Verweilzeit von mindestens 15 Minuten, d. h. mindestens 5 weitere Reaktionsböden bei einer Integration in die Veresterungskolonne oder einen entsprechend dimensionierten Reaktor, z. B. eine Rührkesselkaskade oder einen Rohrreaktor.

Die entsprechende Gesamtbilanz für den Prozeß bei 90%igem Umsatz in der Veresterungskolonne kann dem Blockfließbild 2 (Fig. 4) entnommen werden. Bei sonst gleichen Reaktionsbedingungen muß die Reaktionskolonne um mindestens 10 Böden zur Erzielung des höheren Umsatzes bei Beibehaltung der niedrigen Essigsäurekonzentration im Kopfprodukt verlängert werden. Gegenüber dem 70%igen Umsatz werden jetzt 139 kg/h frischer Essigsäure zur Veresterung verbraucht, während über das Anhydrid nur noch 129 kg/h erzeugt werden, d. h. es wird pro Mol Glycerin bei einem Überschuß von 1,1 Mol Essigsäure verestert. Einmal wird weniger Anhydrid bei der Nachacetylierung verbraucht, außerdem enthält das Sumpfprodukt der Veresterungskolonne bei 90%igen Umsatz nur noch ca. 1% Wasser, so daß eine wesentlich kleinere Wassermenge in der Nachacetylierungsstufe zu Essigsäure umgesetzt zu werden braucht.

Entsprechend dem höheren Umsatz in der Veresterung ist bei sonst gleichen Reaktionsbedingungen gegenüber einem 70%igen Umsatz eine geringere Verweilzeit in der Nachacetylierungsstufe erforderlich; d. h. es sind jetzt nur noch mindestens drei Reaktionsböden bei einer Integration in die Veresterungskolonne oder ein entsprechend dimensionierter Nachreaktor erforderlich.

Es ist eine Frage der Wirtschaftlichkeit, wie der Umsatz auf die Veresterung und Nachacetylierung aufgeteilt wird. Ein Umsatz unter 65—70% in der Veresterung ist unwirtschaftlich, weil dann in der Nachacetylierungsstufe mehr Essigsäure erzeugt als in der Veresterung verbraucht wird, so daß Umsätze von mindestens 70% in der Veresterung anzustreben sind. Höhere Umsätze sind nur durch eine entsprechende Verlängerung der Verweilzeit, d. h. durch mehr Reaktionsböden zu erzielen. Eine Erhöhung des Essigsäureüberschusses bei gleicher Verweilzeit, d. h. z. B. gleicher Bodenzahl, führt nur zu einer unwesentlichen Erhöhung des Umsatzes, verschlechtert jedoch primär das Kopfprodukt durch Erhöhung der Essigsäurekonzentration. Andererseits ist die Reaktionsgeschwindigkeit der Nachacetylierung wesentlich größer als die der Veresterung, so daß für die Acetylierung weniger Reaktionsraum, d. h. weniger Böden erforderlich sind. Auch muß berücksichtigt werden, daß Anhydrid teurer als Essigsäure ist. Es ist letztlich eine Optimierungsfrage, um zu den niedrigsten Herstellkosten unter Berücksichtigung der Rohstoff- und Investitionskosten zu gelangen.

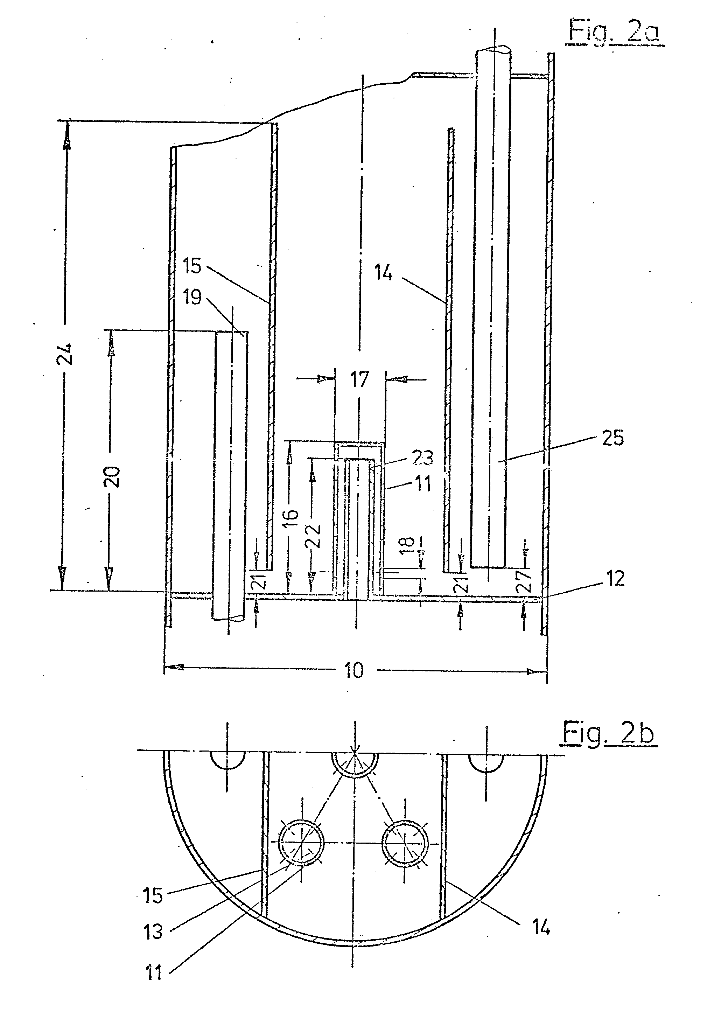

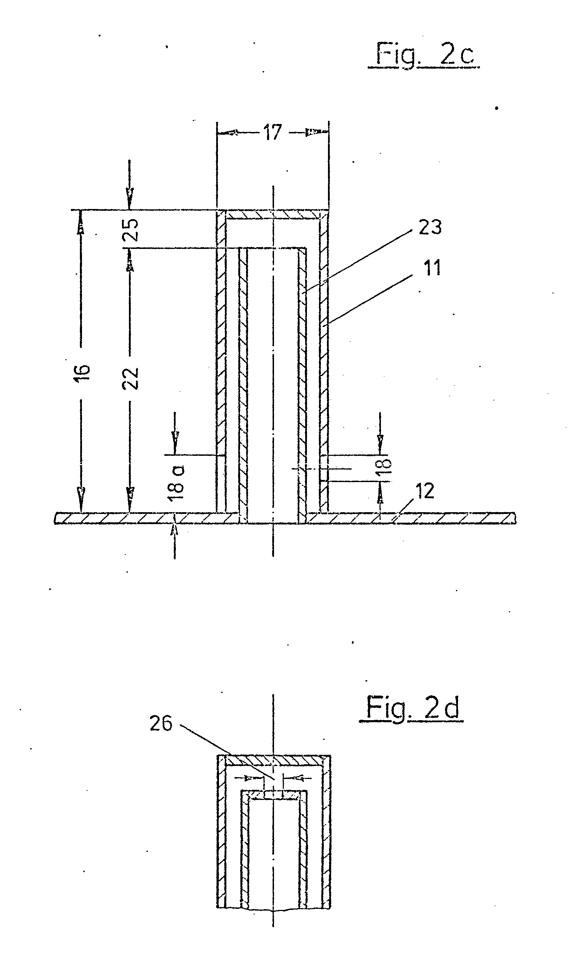

Erfindungsgemäß wird die Veresterung in einer speziellen Glockenbodenkolonne durchgeführt. Es wird konstruktiv eine einwandfreie Funktion des Glockenbodens bei den in der Reaktionskolonne zur Erzielung der erforderlichen Verweilzeit notwendigen hohen Flüssigkeitsständen bei gleichzeitig niedrigen Dampfbelastungen durch das Doppelglockenprinzip erreicht. Die Doppelglocke besteht aus einer inneren Glocke 23 (Fig. 2c) zur Einstellung des trockenen Druckverlustes und einer äußeren Glocke 11 (Fig. 2c), die für die erforderliche Verteilung des Dampfes in der Flüssigkeit sorgt. Die innere Glocke ist auch zum Anfahren der Kolonne erforderlich, um einen Mindestflüssigkeitsstand auf dem Boden so einstellen zu können, daß das Zulaufrohr 26 (siehe Fig. 2a) des darüberliegenden Bodens in der Flüssigkeit abgetaucht ist und so beim Anfahren der Dampf nur durch die Glocken und nicht durch die Zu- und Ablaufrohre der Böden strömen kann.

In einer für die Durchführung der erfindungsgemäßen Verfahren besonders geeigneten Glockenbodenkolonne (Fig. 3) mit mindestens 20 Böden beträgt die gesamte von den Glocken 11 (Fig. 2a, 2a, b, c) auf dem Boden 12 eingenommene Fläche 13 je nach Dampfbelastung 10 bis 30% der zwischen dem Zulaufwehr 14 und dem Ablaufwehr 15 liegenden Bodenfläche. Das Verhältnis der Höhe 16 der Glocken 11 zum Durchmesser 17 der Glocken 11 beträgt 0,5 : 1 bis 3 : 1. In den Glocken 11 sind 4 bis 12 Bohrungen 18 mit Durchmessern von 2 bis 6 mm auf dem Umfang der Glocken 11 gleichmäßig verteilt angeordnet. Diese Bohrungen 18 befinden sich in 5 bis 10 mm Höhe über dem Boden 12. Die Durchtrittsfläche durch die Bohrungen 18 für den Dampf beträgt 0,3 bis 3% bezogen auf den freien Kolonnenquerschnitt. Die Höhe 20 des Ablaufrohres 19 ist größer als 80 mm. Die Höhe 22 der inneren Glocke 23 ist mindestens 20 mm größer als der Abstand 27 des Ablaufrohres 26 vom Boden 12.

In einer alternativen Ausgestaltung der Glocken 11 (Fig. 2c) sind anstelle der Bohrungen 18 in der Glocke 11 in der Nähe des Bodens 12 4 bis 12 senkrechte Schlitze 18a von 2 bis 5 mm Breite und 5 bis 15 mm Höhe vorgesehen.

Die Höhe 24 der Zu- und Ablaufwehre 14, 15 (Fig. 2a) ist größer als die Höhe des Auslaufrohres 19, das den Flüssigkeitsstand auf dem Boden 12 bewirkt. Die Wehre 14, 12 besitzen ferner Schlitze 21 in der Nähe des Bodens 12. Wenn der Bodendurchmesser mehr als 300 mm beträgt, ist in dem Hals der inneren Glocke 23 (Fig. 2d) eine Bohrung 26 von 3 bis 15 mm Durchmesser vorgesehen. Der hierdurch bewirkte trockene Druckverlust ist mindestens doppelt so groß wie der hydrostatische Höhenunterschied der Flüssigkeit zwischen Zulauf und Ablauf und beträgt mindestens 15 bis 20 mm WS.

Eine andere Möglichkeit, den erforderlichen trockenen Druckverlust zu bewirken, ist in Fig. 2c dargestellt. Dort ist der Abstand 25 zwischen der Oberkante des Halses der inneren Glocke 23 und der äußeren Glocke 11 2 bis 6 mm groß.

### Beispiele

### Beispiel 1

Zur Durchführung des Verfahrens diente eine Reaktionskolonne gemäß Fig. 4 mit einem inneren Durchmesser von 350 mm und 32 Glockenböden entsprechend Fig. 2a, 2b und 2c. Das für die flüssige Reaktionsphase zur Verfügung stehende Volumen, bezogen auf die unbegaste Flüssigkeit, betrug 400 l.

Auf die Reaktionskolonne K1 war eine Verstärkungskolonne K2 aufgesetzt, die dazu diente, aus dem Dampf, der von dem obersten Boden der Reaktionskolonne K1 aufsteigt, die Glycerin- und Acetinanteile abzutrennen. Die Verstärkungskolonne K2 wurde mit einem Rücklaufverhältnis von etwa 0,2 so betrieben, daß das Kondensat aus der Verstärkungskolonne K2 nur Reaktionswasser und ca. 2 Gew.-% Essigsäure enthielt.

Bei einem Druck von 7,5 bar wurde auf den obersten Boden der Reaktionskolonne K 1 176 kg/h (1,92 kmol/h) Glycerin der Qualität DAB VII (OH-Zahl 1828) mit einer Temperatur von 250°C aufgegeben. Unter den 32. Boden der Reaktionskolonne K1 wurden 326 kg/h (5,43 kmol/h) auf 250°C erhitzter Essigsäuredampf (Reinheit: 99,5 Gew.-% Essigsäure) eingespeist. Das molare Verhältnis Essigsäure : Glycerin betrug demnach 2,83 : 1. Aus dem Sumpf der Reaktionskolonne K1 wurden 387 kg/h Reaktionsprodukt abgezogen, das zu 75 Gew.-% aus Mono-, Di- und Triacetin bestand und 22 Gew.-% Essigsäure und 3 Gew.-% Wasser enthielt.

Bei den angegebenen Durchsätzen ergibt sich für das flüssige Reaktionsgemisch eine mittlere Verweilzeit von ca. einer Stunde.

Die OH-Zahl des erhaltenen Reaktionsproduktes betrug 549. Der Umsatz, bezogen auf eingesetztes Glycerin, betrug somit 70%. In einem Nachreaktor wurde dieses Reaktionsprodukt mit Essigsäureanhydrid auf eine OH-Zahl von weniger als 0,1 acetyliert.

Das aus dem Kondensator der Verstärkungskolonne K2 mit einem Mengenstrom von 40 kg/h ablaufende Destillat enthielt 98 Gew.-% Wasser und 2 Gew.-% Essigsäure. Daneben waren noch Spuren von Glycerin und Acetin vorhanden.

### Beispiel 2

Analog zu Beispiel 1 wurde in einer Kolonne mit 48 Reaktionsböden ein Sumpfprodukt mit einer OH-Zahl = 91,5 erzielt, d. h. ein Umsatz von 95% erreicht. Hierzu wurden wieder 176 kg/h Glycerin auf den obersten Boden der Veresterungskolonne aufgegeben. Unter dem 48. Boden wurden 463 kg/h (7,72 kmol/h) überhitzter Essigsäuredampf mit einer Temperatur von 250°C eingespeist, d. h. das molare Verhältnis Essigsäure : Glycerin betrug 4,01 : 1. Der Druck in der Kolonne betrug wiederum 7,5 bar, die Reaktionstemperatur 250°C.

Aus dem Sumpf der Veresterungskolonne wurden 442 kg/h Sumpfprodukt abgezogen, welches zu 69,3 Gew.-% aus Di- und Triacetin bestand und 30 Gew.-% Essigsäure und 0,7 Gew.-% Wasser enthielt. In einem Nachreaktor wurde dieses Gemisch mit Essigsäureanhydrid auf eine OH-Zahl <0,1 acetyliert.

Der aus der Kolonne K2 abgezogene Destillatmengenstrom von 101 kg/h bestand im wesentlichen aus Wasser und enthielt ca. 3 Gew.-% Essigsäure und Spuren von Glycerin und Acetin.

### Beispiel 3

Wie in Beispiel 1 wurde bei einem Druck von 7,5 bar und einer Reaktionstemperatur von 250°C in einer Kolonne mit 32 Böden verestert. Auf den obersten Boden wurde 176 kg/h (1,92 kmol/h) Glycerin aufgegeben, während unter den untersten Boden 326 kg/h (5,43 kmol/h) überhitzter Essigsäuredampf von 250°C und auf den untersten Boden 166 kg/h (1,62 kmol/h) Essigsäureanhydrid eingespeist wurden. Außerdem wurden in den der Veresterungskolonne nachgeschalteten Nachreaktor noch 100 kg/h (0,97 kmol/h) Anhydrid zugeführt. Die in der Zeiteinheit zugeführte Essigsäureanhydridmenge war so bemessen, daß einerseits die Gesamtmenge des Wassers das beim 70%igen Umsatz nach Beispiel 1 im Sumpfprodukt gelöst war und die andererseits aus der Veresterung der noch vorhandenen freien OH-Gruppen des Glycerins zu erwarten war, quantitativ zu Essigsäure abreagieren konnte. Außerdem ist in die Anhydridmenge ein Überschuß von 10% eingerechnet, der direkt oder nach einer Abtrennung von der Essigsäure in einer Aufbereitungsanlage zurückgeführt wird.

Das molare Verhältnis Essigsäure : Glycerin betrug 2,83 : 1, das entsprechende von Essigsäureanhydrid : Glycerin = 1,35 : 1.

Das aus dem Sumpf der Reaktionskolonne abgezogene Reaktionsgemisch hatte eine OH-Zahl von 74, d. h. bezogen auf das Glycerin wurde ein Umsatz von 96% erzielt. Diesem Reaktionsgemisch wurden 100 kg/h Essigsäureanhydrid zugemischt und einem Rohrreaktor als Nachreaktor zugeführt, in dem die Reaktion auf eine OH-Zahl <0,1 ablief.

Am Kopf der Kolonne K2 wurde das Reaktionswasser abgezogen, welches noch 2,5 Gew.-% Essigsäure enthielt und nach der Neutralisation in eine biologische Kläranlage geleitet wurde.

### Beispiel 4

In einer Reaktionskolonne wie im Beispiel 1 jedoch mit 42 Böden wurde bei einem Druck von 7,5 bar und einer Reaktionstemperatur von 250°C auf dem obersten Boden 176 kg/h (1,92 kmol/h) Glycerin aufgegeben, welches mit dem unter den 32. Boden eingespeisten 326 kg/h (5,43 kmol/h) überhitzten Essigsäuredampf und dem auf den 32. Boden aufgegebenen 266 kg/h (2,59 kmol/h) auf Reaktionstemperatur vorgewärmten Essigsäureanhydrid reagierte. Das molare Verhältnis Essigsäure : Glycerin betrug 2,83 : 1, das entsprechende von Essigsäureanhydrid : Glycerin = 1,35 : 1.

Die in der Zeiteinheit zugeführte Essigsäureanhydridmenge war so bemessen, daß einerseits die Gesamtmenge des Wassers, die im Reaktionsgemisch auf den 32. Boden gelöst war und die andererseits aus der Veresterung der noch vorhandenen freien OH-Gruppen des Glycerins zu erwarten war, quantitativ zu Essigsäure abreagieren konnte. Außerdem ist in die Anhydridmenge ein Überschuß von 10% eingerechnet, der direkt oder nach einer Abtrennung von der Essigsäure in einer Aufbereitungsanlage in den Prozeß zurückgeführt wird.

Aus dem Sumpf der Kolonne wurde ein Reaktionsgemisch abgezogen, welches neben Essigsäure und Essigsäureanhydrid Triacetin mit einer OH-Zahl <0,1 enthielt. Der Sumpf der Kolonne war als Verdampfer ausgeführt, wodurch ein Teil der im Sumpf gelösten Essigsäure verdampft wurde, die dann in der Kolonne nach oben stieg und für die notwendige Durchmischung auf den Böden 33 bis 42 sorgte.

Am Kopf der Kolonne K2 wurde das Reaktionswasser abgezogen, welches noch 2,5 Gew.-% Essigsäure enthielt. Nach einer Neutralisation wurde das Kopfprodukt in eine biologische Kläranlage geleitet.

### Beispiel 5

In einer Reaktionskolonne wie im Beispiel 1 und 4 jedoch mit 54 Reaktionsböden wurden auf dem obersten Boden 176 kg/h (1,92 kmol/h) Glycerin aufgegeben. Unter den 48. Boden wurden 463 kg/h (7,72 kmol/h) überhitzter Essigsäuredampf eingespeist. Auf dem 48. Boden wurden dem Reaktionsgemisch 41 kg/h (0,40 kmol/h) auf Reaktionstemperatur vorgewärmtes Essigsäureanhydrid zugemischt. Das molare Verhältnis Essigsäure : Glycerin betrug 4,02 : 1, das entsprechende von Essigsäureanhydrid : Glycerin = 0,21 : 1. Die in der Zeiteinheit zugeführte Essigsäureanhydridmenge war so bemessen, daß einerseits die Gesamtmenge des Wassers, die im Reaktionsgemisch auf dem 48. Boden gelöst war und die andererseits aus der Veresterung der noch vorhandenen freien OH-Gruppen des Glycerins zu erwarten war, quantitativ zu Essigsäure abreagieren konnte. Außerdem ist in die Anhydridmenge ein Überschuß von 10% eingerechnet, der direkt oder nach einer Abtrennung von der Essigsäure in einer Aufbereitungsanlage in den Prozeß zurückgeführt wird.

Aus dem Sumpf der Kolonne wurde ein Reaktionsgemisch abgezogen, welches Triacetin mit einer OH-Zahl <0,1 neben Essigsäure und Essigsäureanhydrid enthielt. Der Sumpf der Kolonne war als Verdampfer ausgeführt, wodurch ein Teil der im Sumpf gelösten Essigsäure verdampft wurde, die dann in der Kolonne nach oben stieg und für die notwendige Durchmischung auf den Böden 49 bis 54 sorgte.

Am Kopf der Kolonne K2 wurde das Reaktionswasser abgezogen, welches noch 2,5 Gew.-% Essigsäure enthielt. Nach einer Neutralisation wurde das Kopfprodukt in eine biologische Kläranlage geleitet.

## Beispiel 6

Analog zum Beispiel 1 wurde bei einem Druck von 1,5 bar auf den obersten Boden der Reaktionskolonne K1 167 kg/h (1,92 Kmol/h) Glycerin der Qualität DAB VII (OH-Zahl 1828) mit einer Temperatur von 150° C aufgegeben. Das Glycerin enthielt 0,5 Gew.-% p-Toluolsulfonsäure als Katalysator. Unter dem 32. Boden der Reaktionskolonne K1 wurden 326 kg/h (6,43 kmol/h) auf 150° C erhitzter Essigsäuredampf (Reinheit 99,5 Gew.-% Essigsäure) eingespeist. Das molare Verhältnis Essigsäure : Glycerin betrug demnach 2,83 : 1. Aus dem Sumpf von K1 wurde Rohacetin mit einer OH-Zahl von 311, entsprechend einem Umsatz bezogen auf das Glycerin von 83%, abgezogen. Das Sumpfprodukt enthielt neben 24% Essigsäure noch etwa 1% Wasser.

In einem Nachreaktor wurde das Sumpfprodukt aus K1 mit Essigsäureanhydrid auf eine OH-Zahl <0,1 acetyliert.

Das aus dem Kondensator der Verstärkungskolonne K2 ablaufende Destillat enthielt 98 Gew.-% Wasser und 2 Gew.-% Essigsäure. Daneben waren noch Spuren von Glycerin und Acetin nachweisbar.

## Patentansprüche

1. Verfahren zur Herstellung von Triacetin durch Umsetzung von Glycerin mit Essigsäure und Essigsäureanhydrid bei erhöhter Temperatur und gegebenenfalls in Anwesenheit von Katalysatoren, dadurch gekennzeichnet, daß man die Reaktionspartner in kontinuierlicher Arbeitsweise im Gegenstrom miteinander umsetzt, wobei man in einer mit einer Mehrzahl von Böden ausgestatteten Veresterungskolonne bei einem Druck von 0,2 bis 30 bar und einer Temperatur von 100 bis 250° C flüssiges Glycerin einem aufsteigenden Strom von überhitztem Essigsäuredampf entgegenführt, wobei die Verweilzeit des flüssigen Reaktionsgemisches mindestens eine Stunde beträgt, und dem herabströmenden Reaktionsgemisch beim Erreichen einer OH-Zahl von weniger als 600 auf dem entsprechenden Boden der Reaktionskolonne oder in einem entsprechend ausgelegten Nachreaktor soviel Essigsäureanhydrid zuführt, daß das an dieser Stelle in der flüssigen Reaktionsphase gelöste Wasser quantitativ zu Essigsäure und das vorhandene Mono- und Diacetin quantitativ zu Triacetin abreagieren können.

2. Verfahren nach Anspruch 1, dadurch gekennzeichnet, daß man die Umsetzung in Abwesenheit von Katalysatoren bei einem Druck von 3 bis 30 bar und einer Temperatur von 180 bis 250° C durchführt.

3. Verfahren nach Anspruch 1, dadurch gekennzeichnet, daß man die Umsetzung in Anwesenheit von Katalysatoren bei einem Druck von 0,2 bis 3 bar und einer Temperatur von 100 bis 180° C durchführt.

4. Verfahren nach Anspruch 3, dadurch gekennzeichnet, daß man die Umsetzung in Gegenwart von sauren Veresterungskatalysatoren durchführt, wobei die Katalysatormenge, bezogen auf eingesetztes Glycerin, 0,01 bis 0,5 Gewichtsprozent beträgt.

5. Verfahren nach den Ansprüchen 3 und 4, dadurch gekennzeichnet, daß man die Umsetzung in Gegenwart von p-Toluolsulfonsäure durchführt.

6. Verfahren nach den Ansprüchen 1 bis 5, dadurch gekennzeichnet, daß man als Nachreaktor einen Rohrreaktor oder eine Rührkesselkaskade verwendet.

7. Verfahren nach den Ansprüchen 1 bis 6, dadurch gekennzeichnet, daß man Essigsäure in einem molaren Verhältnis vom Glycerin von 2,5 : 1 bis 5 : 1 einsetzt.

8. Verfahren nach den Ansprüchen 1 bis 7, dadurch gekennzeichnet, daß man dem Reaktionsgemisch pro Mol umzusetzendem Glycerin 0,1 bis 1,5 Mol Essigsäureanhydrid zuführt.

9. Verfahren nach den Ansprüchen 1 bis 8, dadurch gekennzeichnet, daß die Verweilzeit des Reaktionsgemisches auf soviele Böden verteilt wird, daß im Sumpf der Kolonne ein Reaktionsprodukt mit einer OH-Zahl von weniger als 600 und am Kopf der Kolonne das Reaktionswasser mit maximal 3 Gew.-% Essigsäure abgezogen werden kann.

10. Verfahren nach den Ansprüchen 1 bis 9, dadurch gekennzeichnet, daß es in einer Glockenbodenkolonne mit mindestens 20 Doppelglockenböden durchgeführt wird, wobei

a) die gesamte, von den äußeren Glocken 11 auf einem Boden 12 eingenommene Fläche 13 10 bis 30% der zwischen dem Zulaufwehr 14 und dem Ablaufwehr 15 liegenden Fläche des Bodens 12 beträgt,

b) das Verhältnis der Dampfdurchtrittsfläche durch die auf den Boden 12 aufgesetzten äußeren Glocken 11, bezogen auf den freien Kolonnenquerschnitt 0,3 bis 3% beträgt, die auf mindestens 4 Bohrungen 18 pro Glocke 11 mit Durchmesser von 2 bis 5 mm verteilt wird, welche sich auf dem Umfang der Glocken 11 in 5 bis 10 mm Höhe über dem Boden 12 befinden.

c) die Höhe 22 der inneren Glocke 23 mindestens 20 mm größer ist als der Abstand 27 des Ablaufwehres 26 vom Boden 12.

11. Verfahren nach Anspruch 10, dadurch gekennzeichnet, daß an Stelle der Bohrungen 18 in der äußeren Glocke 11 in der Nähe des Bodens 12 4 bis 12 senkrechte Schlitze 18a von 2 bis 5 mm Breite und 5 bis 15 mm Höhe vorhanden sind.

12. Verfahren nach den Ansprüchen 10 und 11, dadurch gekennzeichnet, daß in dem Hals der inneren Glocke 23 eine Bohrung 26 von 3 bis 15 mm Durchmesser vorhanden ist.

13. Verfahren nach den Ansprüchen 10 und 11, dadurch gekennzeichnet, daß der Abstand 25 zwischen der Oberkante des Halses der inneren Glocke 23 und der äußeren Glocke 11 2 bis 6 mm beträgt.

## Claims

1. A process for the production of triacetin by reacting glycerol with acetic acid and acetic acid anhydride at elevated temperature and optionally in the presence of catalysts, characterised in that the reactants are continuously reacted with one another in countercurrent, liquid glycerol being passed in countercurrent to an ascending stream of superheated acetic acid vapour through a multiple-plate esterification column under a pressure of from 0.2 to 30 bars and at a temperature of from 100 to 250°C, the residence time of the liquid reaction mixture amounting to at least one hour and acetic acid anhydride being fed into the descending reaction mixture on reaching an OH-number below 600 either on the corresponding plate of the reaction column or in a correspondingly constructed after-reactor in such a quantity that the water dissolved in the liquid reaction phase at this point can react off quantitatively to form acetic acid whilst the mono- and diacetin present can react off quantitatively to form triacetin.

2. A process as claimed in Claim 1, characterised in that the reaction is carried out in the absence of catalysts under a pressure of from 3 to 30 bars and at a temperature of from 180 to 250°C.

3. A process as claimed in Claim 1, characterised in that the reaction is carried out in the presence of catalysts under a pressure of from 0.2 to 3 bars and at a temperature of from 100 to 180°C.

4. A process as claimed in Claim 3, characterised in that the reaction is carried out in the presence of acid esterification catalysts used in a quantity of from 0.01 to 0.5% by weight, based on the glycerol used.

5. A process as claimed in Claims 3 and 4, characterised in that the reaction is carried out in the presence of p-toluene sulfonic acid.

6. A process as claimed in Claims 1 to 5, characterised in that a tube reactor or a cascade of stirrer-equipped vessels is used as the after-reactor.

7. A process as claimed in Claims 1 to 6, characterised in that acetic acid is used in a molar ratio to the glycerol of from 2.5 : 1 to 5 : 1.

8. A process as claimed in Claims 1 to 7, characterised in that from 0.1 to 1.5 moles of acetic acid anhydride are fed into the reaction mixture per mole of glycerol to be reacted.

9. A process as claimed in Claims 1 to 8, characterised in that the residence time of the reaction mixture is spread over such a number of plates that a reaction product having an OH-number of less than 600 can be run off from the sump of the column whilst the water of reaction containing at most 3% by weight of acetic acid can be run off at the head of the column.

10. A process as claimed in Claims 1 to 9, characterised in that it is carried out in a bubble plate column comprising at least twenty double bubble plates,

a) the total are a 13 occupied by the outer bubble caps 11 on one plate 12 amounting to between 10 and 30% of the area of the plate 12 situated between the inflow weir 14 and the outflow weir 15,

b) the vapour throughflow area through the outer bubble caps 11 on the plate 12 making up from 0.3 to 3% of the free column cross-section spread over at least 4 bores 18 from 2 to 5 mm in diameter per bubble cap 11 which are situated at the periphery of the bubble caps 11 from 5 to 10 mm above the plate 12,

c) the height 22 of the inner bubble cap 23 is at least 20 mm greater than the distance 27 between the outflow weir 26 and the plate 12.

11. A process as claimed in Claim 10, characterised in that from 4 to 12 vertical slots 18a from 2 to 5 mm wide and from 5 to 15 mm tall are present instead of the bores 18 in the outer bubble cap 11 in the vicinity of the plate 12.

12. A process as claimed in Claims 10 and 11, characterised in that a bore 26 from 3 to 15 mm in diameter is present in the neck of the inner bubble cap 23.

13. A process as claimed in Claims 10 and 11, characterised in that the distance 25 between the upper edge of the neck of the inner bubble cap 23 and the outer bubble cap 11 is between 2 and 6 mm.

## Revendications

1. Procédé de fabrication de triacétine par réaction de la glycérine avec de l'acide acétique et de l'anhydride acétique à température élevée et éventuellement en présence de catalyseurs, caractérisé en ce qu'on fait réagir ensemble les partenaires de réaction suivant un mode opératoire continu en contre-courant, en faisant passer dans une colonne d'estérification comportant une multiplicité de plateaux, sous une pression de 0,2 à 30 bars et à une température de 100 à 250°C, de la glycérine liquide en sens contraire d'un courant ascendant de vapeur surchauffée d'acide acétique, le temps de séjour du mélange de réaction liquide s'élevant au moins à une heure, et en ajoutant au mélange de réaction descendant, quand il atteint un indice OH inférieur à 600, sur le plateau approprié de la colonne de réaction ou dans un post-réacteur adapté en rapport, de l'anhydride acétique en quantité suffisante pour que l'eau dissoute à cet endroit dans la phase de réaction liquide soit convertie quantitativement en acide acétique et pour que la mono- et diacétine présentes puissent réagir quantitativement en triacétine.

2. Procédé selon la revendication 1, caractérisé en ce qu'on effectue la réaction en l'absence de catalyseurs sous une pression de 3 à 30 bars et à une température de 180 à 250°C.

3. Procédé selon la revendication 1, caractérisé en ce qu'on effectue la réaction en présence de catalyseurs sous une pression de 0,2 à 3 bars et à une température de 100 à 180°C.

4. Procédé selon la revendication 3, caractérisé en ce qu'on effectue la réaction en présence de catalyseurs acides d'estérification, la quantité de catalyseur par rapport à la glycérine mise en jeu s'élevant à 0,01 à 0,5% en poids.

5. Procédé selon les revendications 3 et 4, caractérisé en ce qu'on effectue la réaction en présence d'acide p-toluène-sulfonique.

6. Procédé selon les revendications 1 à 5, caractérisé en ce qu'on utilise comme post-réacteur un réacteur tubulaire ou une cascade de cuves à agitation.

7. Procédé selon les revendications 1 à 6, caractérisé en ce qu'on utilise l'acide acétique en une proportion molaire par rapport à la glycérine de 2,5 : 1 à 5 : 1.

8. Procédé selon les revendications 1 à 7, caractérisé en ce qu'on alimente au mélange de réaction par mole de glycérine à faire réagir 0,1 à 1,5 mole d'anhydride acétique.

9. Procédé selon les revendications 1 à 8, caractérisé en ce que le temps de séjour du mélange de réaction est réparti sur un nombre tel de plateaux que dans la base de la colonne on puisse soutirer un produit de réaction ayant un indice OH inférieur à 600, et, en tête de la colonne, l'eau de réaction contenant un maximum de 3% en poids d'acide acétique.

10. Procédé selon les revendications 1 à 9, caractérisé en ce qu'on l'exécute dans une colonne à plateaux et à cloches comportant au moins 20 plateaux à cloches doubles, ici

a) la surface totale 13 occupée par les cloches externes 11 sur un plateau 12 s'élevant à 10—30% de la surface du plateau 12 se trouvant entre le barrage d'alimentation 14 et le barrage de décharge 15,

b) le rapport de la surface de passage de la vapeur à travers les cloches externes 11 placées sur le plateau 12, rapportée à la section transversale libre de la colonne s'élevant à 0,3 à 3%, surface qui est répartie sur au moins 4 forages 18 par cloche 11 qui ont un diamètre de 2 à 5 mm et qui se trouvent sur le pourtour des cloches 11 à une hauteur de 5 à 10 mm au-dessus du plateau 12,

c) la hauteur 22 de la cloche interne 23 étant au moins 20 mm plus grande que l'écartement 27 du barrage de décharge 26 par rapport au plateau 12.

11. Procédé selon la revendication 10, caractérisé en ce qu'au lieu des forages 18 sont prévues dans la cloche externe 11 au voisinage du plateau 12 4 à 12 fentes verticales 18a de 2 à 5 mm de large et de 5 à 15 mm de haut.

12. Procédé selon les revendications 10 et 11, caractérisé en ce qu'on prévoit dans le col de la cloche interne 23 un forage 26 de 3 à 15 mm de diamètre.

13. Procédé selon les revendications 10 et 11, caractérisé en ce que l'écartement 25 entre l'arête supérieure du col de la cloche interne 23 et la cloche externe 11 s'élève à 2—6 mm.

Fig. 1

Fig. 2a

Fig. 2b

Fig. 2c

Fig. 2d

**Fig. 3**

Blockfließbild 1

0 033 929

# Fig.4

## Blockfließbild 2

2,78 kmol/h = 50 kg/h Wasser
0,033 kmol/h = 2 kg/h Essigsäure

1,088 kmol/h
100 kg/h
Glyzerin

| Veresterung |

2,32 kmol/h
139 kg/h
Essigsäure

2,15 kmol/h
129 kg/h
Essigsäure

223 kg/h Acetin (OH-Zahl = 183)
90 kg/h Essigsäure
3 kg/h $H_2O$

0,49 kmol/h
50 kg/h
Essigsäure -
anhydrid

| Nachacetylierung |

0,049 kmol/h
5 kg/h
Essigsäure -
anhydrid

237 kg/h Triacetin
129 kg/h Essigsäure
5 kg/h Anhydrid

| Aufarbeitung |

237 kg/h Triacetin
1,088 kmol/h